# EUROPÄISCHE PATENTANMELDUNG

(11) **EP 1 972 215 A1**
(43) Veröffentlichungstag der Anmeldung: **24.09.2008**
(21) Anmeldenummer: 07104524.9
(22) Anmeldetag: 20.03.2007
(51) Int. Cl.: A24F 47/00

(54) **Rauchfreies Zigarettenersatzprodukt**

(71) Anmelder: Wedegree GmbH, 20357 Hamburg (DE)
(72) Erfinder: Rinker, Arno, 20255 Hamburg (DE)
(74) Vertreter: Kihn, Pierre Emile Joseph

(57) **Zusammenfassung**

Vorrichtung in Form und Abmessungen einer Zigarette oder Zigarre zur Abgabe eines inhalierbaren Aerosols, umfassend ein einen Wirk- und/oder Aromastoff-Depot (31) enthaltendes Mundstück (3), einen Heizstab (2) mit einer Gehäusehülse mit einem oder mehreren Lufteingängen und einem oder mehreren mundstückseitigen Warmluftausgängen, einem Füllventil (27) zum Befüllen eines Gastanks (21) mit einem brennbaren Gas, vorzugsweise Propan- oder Butangas, einem Reglerventil (23) zur gesteuerten Abgabe des Gases aus dem Gastank (21) an einen Brenner (24) und einem Wärme- und/oder Stofftauscher (25) zur Erwärmung der Luft durch die mittels des Brenners (24) erzeugten Hitze, wobei das Mundstück (3) mit dem Heizstab (2) lösbar verbunden ist und die Steuerung des Reglerventils (23) mittels des durch den Sog eines Benutzers am Mundstück (3) erzeugten Unterdrucks und/oder Luftstroms erfolgt. Tankstation für eine solche Vorrichtung, sowie Ventil für Inhaliervorrichtung, steuerbar mittels eines durch den Sog eines Benutzers erzeugten Unterdrucks und/oder Luftstroms.

## Beschreibung

### Technisches Gebiet

Die vorliegende Erfindung betrifft allgemein ein rauchfreies Zigarettenersatzprodukt.

### Stand der Technik

Beim Rauchen einer herkömmlichen Zigarette wird Tabak verbrannt und der bei dieser Verbrennung entstehende Rauch wird inhaliert (Hauptstromrauch) oder an die Umgebung abgegeben (Nebenstromrauch).

Der Hauptstromrauch ist maßgeblich für die Gesundheitsschädigung des Konsumenten verantwortlich, liefert ihm aber den gewünschten Genuss. Der Nebenstromrauch ist maßgeblich für die Gesundheitsschädigung der Passivraucher verantwortlich und ist weder von den Passivrauchern noch vom Konsumenten gewünscht.

Im Rauch einer Zigarette wurden über 4800 verschiedene Substanzen nachgewiesen von denen ungefähr 70 als nachweislich krebserregend gelten.

Bis heute existiert jedoch keine ernstzunehmende Konkurrenz zur Zigarette. Die meisten bekannten Produkte sind eigentlich Nikotin-Substitute und sind nicht als Genussprodukte, sondern als Entwöhnungsprodukte gedacht.

Dagegen sind bekannte Produkte die gezielt auf eine Alternative zur Zigarette ohne dessen negativen Aspekte hingerichtet sind oft durch Nachteile behaftet die deren Akzeptanz beim Raucher stark beschränkt.

Gemein ist diesen Produkten, dass sie nicht die Verbrennungswärme des Tabaks, sondern andere Energiequellen zur Freisetzung eines (Rauch)Aerosols benutzen, wodurch die Hauptquelle des Nebenstromrauchs weitgehend entfällt.

Vorrichtungen deren Substrate mittels elektrischer Energie, sei es in Form eines Heizwiderstandes, einer Induktionsheizung oder eines Ultraschallzerstäubers, verflüchtigt werden sind bekannt, haben aber als Nachteil, dass sie wegen ihrer Größe oder ihres Gewichts in der Hand gehalten werden müssen, dass sie sehr teuer in der Herstellung sind und/oder dass sie durch ihre (Schwermetall)Bestandteile nur schwer umweltgerecht zu entsorgen sind.

Auch wurden Produkte vorgestellt deren Energie aus brennbaren kohlenstoffhaltigen Feststoffen, wie Celluloseschäume, Holzkohle, usw. gewonnen wird. Der Nachteil ist hierbei generell, dass ein Nebenstromrauch entsteht wenn auch in geringeren Mengen und im Prinzip weniger mit Schadstoffen belastet als bei herkömmlichen Zigaretten.

### Aufgabe der Erfindung

Eine Aufgabe der vorliegenden Erfindung ist es für den Benutzer den Rauchakt einer normalen Zigarette so vollständig wie möglich zu imitieren. Die haptische Wahrnehmung des Produktes sollte in der Hand wie am Mund durch Größe und Gewicht einer Zigarette oder (kleinen) Zigarre sehr ähnlich sein.

### Allgemeine Beschreibung der Erfindung

Diese Aufgabe wird erfindungsgemäß gelöst durch eine Vorrichtung in Form und Abmessungen einer Zigarette oder Zigarre zur Abgabe eines inhalierbaren Aerosols, umfassend
➢ ein einen Wirk- und/oder Aromastoff-Depot (31) enthaltendes Mundstück (3),
➢ einen Heizstab (2) umfassend
   eine Gehäusehülse mit einem oder mehreren Lufteingängen und
   einem oder mehreren mundstückseitigen Warmluftausgängen,
   ein Füllventil (27) zum Befüllen eines Gastanks (21) mit einem brennbaren Gas, vorzugsweise Propan- oder Butangas,
   ein Reglerventil (23) zur gesteuerten Abgabe des Gases aus dem Gastank (21) an einen Brenner (24) und
   einen Wärme- und/oder Stofftauscher (25) zur Erwärmung der Luft durch die mittels des Brenners (24) erzeugten Hitze,
wobei das Mundstück (3) mit dem Heizstab (2) lösbar verbunden ist und die Steuerung des Reglerventils (23) mittels des durch den Sog eines Benutzers am Mundstück (3) erzeugten Unterdrucks und/oder Luftstroms erfolgt.

Die Vorteile einer solchen Vorrichtung sind vielfältig. Es erfolgt erstens keine Rauchbelästigung durch Verbrennung komplexer Stoffe, wie Tabak, und zweitens entsteht wegen der extrem sauberen Verbrennung eines brennbaren Gases kein sogenannter Nebenstromrauch, wodurch Dritte nicht durch Passivrauchen geschädigt werden. Drittens, dadurch dass im Gegensatz zu einer herkömmlichen Zigarette die Wirk- und/oder Aromastoffe und deren Anzahl gezielt ausgewählt werden können ist deren Inhalation gesundheitlich bei weitem unbedenklicher als bei einer normalen Zigarette und so kann eine krebserregende (karzinogene) Wirkung gezielt ausgeschlossen werden. Anders ausgedrückt, da keine Verbrennung im herkömmlichen Sinn erfolgt, wird eine kontrollierte Aerosolzusammensetzung ermöglicht ohne Dritte zu belästigen.

Im Gegensatz zu bekannten Vorrichtungen die auf einer elektrischen Energiequelle zur Aerosolbildung basieren, benötigt die erfindungsgemäße Vorrichtung keinen Hochleistungsakku, was andererseits niedrigere Herstellungskosten ermöglicht und erheblich weniger Entsorgungsprobleme bereitet.

Weiterhin stellt brennbares Gas, d.h. z.B. sogenanntes Feuerzeuggas, vorzugsweise Propan- und/oder Butangas, als Energiequelle einen weiteren Vorzug der Erfindung dar, da hiermit gute Heizwerte und hervorragende Umweltverträglichkeit kombinierbar sind. Außerdem erlaubt die Wahl dieser Energiequelle auf bereits existierende Infrastrukturen und Erfahrungen zurückzugreifen, was die Herstellungskosten erheblich senkt.

Darüber hinaus ermöglicht die Form und die Abmessungen der Vorrichtung eine Benutzung die der einer herkömmlichen Zigarette sehr ähnlich ist. In diesem Zusammenhang, bedeutet der Ausdruck "Form und Abmessungen einer Zigarette oder Zigarre", dass die Vorrichtung eine grundsätzlich zylindrische Form hat und einen Durchmesser zwischen ca. 6 bis ca. 14 mm, bevorzugt zwischen ca. 8 und 11 mm und eine Länge von ca. 5 bis ca. 15 cm, bevorzugt zwischen ca. 7,5 und ca. 10,5 cm aufweist. Die einfache Handhabung durch den Benutzer wird außerdem durch ein im Vergleich zu bekannten Vorrichtungen sehr geringes Gewicht unterstützt, sodass die Vorrichtung auch im Mund problemlos tragbar ist ohne dass sie permanent in der Hand gehalten werden muss.

Dieser für die Akzeptanz vom Benutzer entscheidende Vorteil wird durch die Verflüchtigung der Wirk- und/oder Aromastoffe (Bildung eines inhalierbaren Aerosols) ausschließlich durch die erwärmte Luft aus dem Wärme- und/oder Stofftauscher erreicht, d.h. ohne das Depot durch direkten Kontakt mit der Energiequelle zu erhitzen. Dabei ist ein inhalierbares Aerosol im Sinne dieser Erfindung im Prinzip ein Gemisch aus festen und/oder flüssigen Schwebeteilchen und Luft. Bevorzugt handelt es sich beim inhalierbaren Aerosol um einen Nebel, d.h. ein Gemisch bestehend hauptsächlich aus flüssigen Schwebeteilchen und Luft, vorzugsweise weitestgehend frei von Feinstaub.

Die Gemeinsamkeiten zur Handhabung einer Zigarette werden noch zusätzlich dadurch verstärkt, dass das Anzünden (d.h. das Starten der Verbrennung, bzw. Beginn des Rauchakts) der erfindungsgemäßen Vorrichtung durch einen Sog durch den Benutzer an der Vorrichtung und gleichzeitiges Halten der dem Mundstück abgewandten Seite an eine Flamme, z.B. von einem Feuerzeug (oder einer einem Feuerzeug im Aussehen und Benutzung sehr ähnlichen Tankstation wie weiter unten beschrieben), erfolgt.

Im Gegensatz zu bekannten Produkten ist also kein manueller Schalter notwendig um die Energiequelle zu starten und durch die Wahl von brennbarem Gas als Energiequelle ist wegen dessen hohem Heizwert eine Gastankfüllung ausreichend für die Dauer einer herkömmlichen Zigarette. Anschließend erlischt der Brenner von selbst, was verhindert, dass der Benutzer vergisst die Vorrichtung auszuschalten und so der Sicherheit dient.

Diese dem Anzünden einer Zigarette sehr ähnliche Prozedur wird durch ein Reglerventil ermöglicht das die Gasabgabe an den Brenner freigibt sobald der Benutzer das erste Mal am Mundstück der Vorrichtung zieht, d.h. wenn ein Sog am Mundstück erfolgt durch den ein Unterdruck und/oder Luftstrom innerhalb der Vorrichtung entsteht, öffnet sich das Reglerventil und leitet Gas an den angeschlossenen Brenner.

In einem weiteren Aspekt betrifft die Erfindung deshalb ein Ventil für eine Inhaliervorrichtung, z.B. für eine Vorrichtung wie oben beschrieben, steuerbar mittels eines durch den Sog eines Benutzers erzeugten Unterdrucks und/oder Luftstroms. Dabei umfasst das Ventil einen Regelkörper, sowie Mittel zur Erzeugung einer Rückstellkraft, wobei der Regelkörper einen ersten geschlossenen Zustand, einen zweiten offenen Zustand, sowie einen dritten minimal offenen Zustand aufweist, wobei der Regelkörper mittels des erzeugten Unterdrucks und/oder Luftstroms aus dem ersten geschlossenen Zustand entgegen einer Rückstellkraft in den zweiten offenen Zustand versetzbar ist und die Mittel zur Erzeugung einer Rückstellkraft derart gewählt sind, dass sie den Regelkörper nur in den dritten minimal offenen Zustand zurücksetzen können. Im Notfall kann durch einen vom Benutzer erzeugten Überdruck und/oder einem dem zuvor erwähnten Luftstrom entgegengesetzten Luftstrom der Regelkörper wieder in den ersten geschlossenen Zustand versetzt werden.

Der Vorteil eines solchen Ventils ist, dass es möglich ist, z.B. im Fall der Reglung eines brennbaren Gases, drei grundsätzliche Betriebsarten darzustellen: Aus (geschlossen), Betriebsflamme (offen) und Pilotflamme (minimal offen). Würde der Regelkörper aus dem offenen Zustand durch die Rückstellkraft wieder in den geschlossenen Zustand gebracht würde in einem solchen Fall die Flamme wieder erlöschen. Dies ist jedoch nicht erwünscht, da der Benutzer einer erfindungsgemäßen Vorrichtung mehrere Züge pro Rauchakt macht und nicht jedes Mal die Vorrichtung wieder anzünden will.

Der erste geschlossene Zustand des Ventils, in dem der Ventilkörper z.B. durch Kraftschluss arretiert ist, ermöglicht es z.B. in einer erfindungsgemäßen Vorrichtung, dass kein Gas entweicht solange der Benutzer nicht am Mundstück zieht (saugt), d.h. der Heizstab kann mit Gas gefüllt werden unabhängig ob der Benutzer gleich "rauchen" will oder nicht, z.B. direkt nach dem Rauchakt.

Darüber hinaus, sobald der Benutzer einen genügenden Unterdruck und/oder Luftstrom innerhalb der Vorrichtung erzeugt, wird der Regelkörper dadurch in einen offenen Zustand gebracht und das Gas kann entweichen. Wird das Gas gleichzeitig durch den Benutzer entzündet verbrennt es im Brenner.

Eine Reglung per Luftstrom wird im Prinzip dadurch erreicht, dass am Regelkörper Strömungshindernisse angeordnet sind die sich innerhalb des Luftkanals befinden und so mittels des durch den Sog erzeugten Luftstroms betätigt werden. Eine Reglung per Unterdruck wird dann erreicht, wenn die durch den Sog entstandene Druckdifferenz vor und hinter dem Ventilkörper dazu genutzt wird diesen zu verstellen. Beide Möglichkeiten zur Reglung sind natürlich kombinierbar. Als genügender Luftstrom kann z.B. ein Volumenstrom zwischen 15 und 20 ml/s, typischerweise ca. 17,5 ml/s gelten. Ein entsprechender Unterdruck beträgt z.B. zwischen 50 und 500 mbar, bevorzugt zwischen 100 und 200 mbar.

Damit eine Reglung des Durchflusses erfolgen kann, wird durch den Sog beim Öffnen des Ventils ein Mittel zur Erzeugung einer Rückstellkraft, z.B. eine Feder, gespannt.

Hört der Benutzer auf am Mundstück zu ziehen, wird der Regelkörper durch diese Rückstellkraft zurückgedrückt. Würde der Regelkörper dabei wieder in den geschlossenen Zustand zurückgestellt, würde die Flamme wie bereits erwähnt erlöschen. Das Ventil gemäß der Erfindung ist deshalb derart gestaltet, dass die Mittel zur Erzeugung einer Rückstellkraft so bemessen sind, dass sie den Regelkörper nur in einen dritten Zustand zurückzustellen vermögen bei dem ein minimaler Durchfluss (minimaler Strömungsquerschnitt) des Gases gewährleistet ist.

So ist in diesem Zusammenhang unter "minimal offen" oder den entsprechenden Ausdrücken "minimaler Durchfluss" oder "minimaler Strömungsquerschnitt" ein Zustand zu verstehen in dem eine geringe Menge an Gas durchströmt, die gerade ausreicht damit die Flamme in einem nachgeschalteten Brenner nicht erlischt. Der daraus resultierende Betriebszustand, auch Pilotflamme genannt, ermöglicht es dem Benutzer mehrere Züge zu tätigen ohne jedes Mal neu zu zünden, dabei aber möglichst wenig Gas zu verbrauchen, was einer kompakteren Bauweise wie im Zusammenhang mit der erfindungsgemäßen Vorrichtung zugute kommt.

Bevorzugt, gibt der Regelkörper in dem zweiten offenen Zustand einen Strömungsquerschnitt frei der abhängig vom Unterdruck und/oder Luftstrom variabel zwischen einem minimal offenen bis ganz offenen Zustand steuerbar ist, damit wird erreicht, dass eine feinere Reglung abhängig von der Sogkraft des Benutzers, bzw. dessen Rauchgewohnheiten möglich ist.

Wie schon angedeutet findet ein solches Ventil bevorzugt Anwendung in einer erfindungsgemäßen Vorrichtung zur Abgabe eines inhalierbaren Aerosols als Reglerventil.

In einer weiteren Ausgestaltung dieser Vorrichtung, umfasst diese zusätzlich ein Hauptventil (22) zwischen Gastank (21) und Reglerventil (23) welches durch Verbinden des Mundstücks (3) mit dem Heizstab (2) geöffnet und durch Ablösen des Mundstücks (3) wieder geschlossen wird. Damit wird gewährleistet, dass kein Gas entweichen kann, solange die Vorrichtung nicht betriebsbereit ist, d.h. solange das Mundstück nicht auf den Heizstab aufgesetzt wurde.

Der Brenner des Heizstabs ist vorzugsweise ein Flüssiggas-Vormischbrenner, d.h. das brennbare Gemisch wird bereits vor dem Düsenaustritt gebildet, sodass eine vollständige Verbrennung des Flüssiggases gewährleistet wird. Zu dieser Art von Brennern werden z.B. Nainen-, Ikari- oder Matrix-Brenner gezählt und sind teilweise aus Sturmfeuerzeugen bekannt.

Die Ähnlichkeit zu einer herkömmlichen Zigarette während der Benutzung kann weiterhin vervollständigt werden indem ein Glühen oder Glimmen an der dem Mundstück abgewandten Seite auftritt sobald der Benutzer am Mundstück zieht. Dies wird z.B. erreicht mit einem Metallgitter das in der Flamme oder der Flammfront angeordnet ist. Bei jedem Zug an der Vorrichtung wird so durch die größere und wärmere Betriebsflamme das Gitter zum Glühen gebracht. Das Gitter sollte dabei hochhitzebeständig sein, d.h. nicht bei den in der Flamme erreichten Temperaturen verglühen, beispielweise kann ein solches Gitter aus Wolfram oder einer Wolfram-Legierung bestehen.

Da zum Teil sehr hohe Temperaturen im Betrieb erreicht werden können, kann es sinnvoll sein einen Überhitzungsschutz, z.B. einen Bimetallregler vorzusehen. Dieser kann so gestaltet werden, dass er durch Ausdehnung bei Überhitzung das Reglerventil schließt und so die Gaszufuhr zum Brenner unterbricht. Dies kann z.B. erfolgen indem er durch seine Ausdehnung dem Reglerventil einen zusätzlichen (vierten) Zustand erlaubt in dem das Reglerventil geschlossen ist oder indem er das Reglerventil in seinen ersten Zustand zurückschiebt. Beides kann dadurch erreicht werden, dass die Mittel zur Erzeugung einer Rückstellkraft, z.B. eine Feder, so beeinflusst werden, z.B. durch Verschieben des Federwegs, dass sie das Ventil in eine vierte geschlossene oder, alternativ, in ihre erste geschlossene Position zurücksetzen können.

Die Erwärmung der zur Verflüchtigung der im Depot enthaltenen Substanzen benötigten Luft erfolgt erfindungsgemäß durch einen Wärmetauscher, durch einen Stofftauscher oder einer Kombination daraus. Da eine Temperaturerhöhung der Inhalationsluft z.B. um mindestens 180°C erforderlich ist, damit die Inhalationsluft beim Verlassen des Depots noch eine Temperatur von 100° C besitzt, muss ein reiner Wärmetauscher mit ausreichender Übertragungsfähigkeit vorgesehen werden ohne die geforderten Dimensionen des Heizstabs zu überschreiten.

Es wurde nun festgestellt dass je nach Art der Wirk- und/oder Aromastoffe, d.h. in Abhängigkeit von der benötigten Verflüchtigungswärme, ein solcher Wärmetauscher entweder zu groß wird und/oder einen zu hohen Gasverbrauch mit sich bringt, was wiederum zu einer zu großen Vorrichtung führen würde.

Demgemäss, beinhaltet der Heizstab bevorzugt einen sogenannten Stofftauscher in dem die Abgase der Verbrennung des brennbaren Gases mindestens zum Teil als Warmluft genutzt werden, wobei zur Einstellung der benötigten Temperatur Frischluft hinzu gemischt wird. So können z.B. 3 bis 100 %, vorzugsweise 5 bis 50 % der Abgase in den Warmluftstrom eingeführt werden.

Wegen der kompakten Abmessungen einer erfindungsgemäßen Vorrichtung, ist diese bevorzugt so gestaltet, dass ein oder mehrere zu dem oder den Warmluftausgängen führende Warmluftkanäle koaxial um den Gastank (21) angeordnet sind. Diese Warmluftkanäle führen die erwärmte Luft vom Wärme- und/oder Stofftauscher zu den Warmluftausgängen, wo sie bei aufgesetztem Mundstück durch die Kammern des Depots zwecks Verflüchtigung der darin vorhandenen Wirk- und Aromastoffe zum Mund des Benutzers geführt wird.

Bevorzugt erfolgen beim Genuss eine Anregung der Geschmacksnerven und eine Stimulation der Nikotinrezeptoren. Daher enthält die Vorrichtung in einer bevorzugten Ausführungsform in dem Wirk- und/oder Aromastoff-Depot als Wirkstoff Nikotin und ggf. Aromastoffe, sowie ggf. weitere Zusatz- und Hilfsstoffe, wie Reizstoffe, z.B. Capsaicin, Stabilisatoren z.B. Propylenglykol, Verflüchtigungsvermittler, z.B. Ethanol, usw.

Bei einem Depot mit mehreren Inhaltsstoffen kann es, z.B. aufgrund der unterschiedlichen benötigten Verdampfungsenergien, vorteilhaft oder notwendig sein, dass der oder die Wirkstoff(e), ggf. Aromastoff(e), sowie ggf. weitere Zusatz- und Hilfsstoffe in mehreren Kammern einzeln und/oder gemischt vorliegen. Dabei können die Kammern unterschiedliche Eigenschaften haben in bezug auf Abmessungen und Form um die gewünschte Dosierung der einzelnen Komponenten zu erreichen, z.B. durch Variation des Strömungsquerschnitts und/oder der Depotmaterialien, usw.

Da die oben genannten Gase, wie Butan- und/oder Propan, bei stöchiometrischer Verbrennung, d.h. mit einem λ-Wert (Luftverhältnis) = 1 vollständig zu Kohlenstoffdioxid (CO₂) und Wasser (H₂O) reagieren, ist das Verbrennungsabgas aus gesundheitlicher Sicht sauber. Trotzdem kann es vorkommen, speziell bei Lastwechselreaktionen, dass dieser λ-Wert zeitweise kleiner als 1 ist, d.h. die Verbrennung ist teilweise unvollständig, was in der Praxis im Fall eines Stofftauschers, d.h. wenn ein Teil der Abgase direkt zur Erwärmung durch Vermischen mit Umgebungsluft benutzt werden, geringe Mengen an Kohlenstoffmonoxid (CO) in der Atemluft bedeuten würde.

Deshalb sieht eine weitere Ausführungsform der Vorrichtung vor, in der ein Stofftauscher eingesetzt wird, dass ein Kohlenstoffmonoxid-Oxydationskatalysator, z.B. ein Hopkalit-Katalysator, dem Stofftauscher nachgeschaltet ist, wodurch etwaige geringe Mengen an Kohlenstoffmonoxid in unbedenkliches Kohlenstoffdioxid (CO₂) umgewandelt werden.

Ein weiterer Aspekt der Erfindung ist es eine Tankstation zum Befüllen des Heizstabs einer erfindungsgemäßen Vorrichtung bereitzustellen, welche einen Gasvorratstank, dessen Volumen das Mehrfache des Volumens des Gastanks des Heizstabs beträgt und ein formschlüssig und gasdicht mit dem Füllventil des Heizstabs verbindbares Auslassventil beinhaltet. Diese ermöglicht es dem Benutzer den wiederverwendbaren Heizstab nach Abnahme (bzw. vor Aufsetzen) des Mundstücks mit brennbarem Gas zu füllen.

Bevorzugt dient die Tankstation nicht nur dazu den Heizstab aufzufüllen, sondern auch um ihn bei Nicht-Benutzung aufzubewahren. Deshalb umfasst die Tankstation in einer weiteren Gestaltungsform weiterhin Mittel zur Aufbewahrung eines Heizstabs mit einer das Füllventil zu mindest teilweise umschließende Aufbewahrungstasche zur Aufnahme eines mundstückseitigen Teils des Heizstabs, sowie einem umsetzbaren Deckel der in einer geschlossenen Stellung die entgegengesetzte Seite des Heizstabs überdeckt. Da dieser Deckel die ggf. noch heiße Spitze des Heizstabs überdeckt ist er bevorzugt als Hitzeschild ausgestaltet, z.B. aus einem hitzebeständigem und vorzugsweise isolierendem Material, damit der Benutzer die Tankstation mit eingelegtem Heizstab sofort gefahrlos z.B. in die Tasche stecken kann.

In einer weiteren Ausgestaltung enthält die Tankstation zusätzlich eine Zündvorrichtung, z.B. einen Piezo-Zünder, mit der sich die Vorrichtung aus Heizstab und Mundstück anzünden lässt.

In einer weiteren bevorzugten Ausgestaltung umfasst die Tankstation zusätzlich zu einer solchen Zündvorrichtung weiterhin ein Brennerventil und einen Brenner, z.B. einen Diffusionsbrenner, einen Teilvormischbrenner oder bevorzugt einen Vormischbrenner, wobei die Zündvorrichtung entweder direkt zum Anzünden der Vorrichtung (Heizstab) dient oder aber innerhalb der Tankstation selbst, die in einem solchen Fall ein komplettes Feuerzeug beinhaltet und auch als solches benutzt werden kann, unabhängig vom Heizstab.

Obwohl die Tankstation als Einwegprodukt ausgestaltet werden kann, ist es vorteilhaft wenn sie weiterhin ein Füllventil zum Befüllen des Gasvorratstanks umfasst, das es erlaubt sie nach Entleerung des Vorratstanks wieder zu befüllen und weiter zu verwenden.

Die Erfindung betrifft selbstverständlich auch die einzelnen Komponenten, d.h. den wiederverwendbaren Heizstab, das Einweg-Mundstück, sowie die dazu passende Tankstation. Bevorzugt betrifft die Erfindung auch ein Kit umfassend eine Tankstation und einen Heizstab wie oben beschrieben, gegebenenfalls mit einer Vielzahl an Mundstücken.

Ein weiterer Aspekt der Erfindung ist die Bereitstellung eines Verfahrens zur Verflüchtigung von Wirk- und Aromastoffen zwecks Inhalation,
wobei Verbrennungsgase eines brennbaren Gases teilweise oder vollständig, gegebenenfalls vermischt mit Umgebungsluft, durch einen Wirk- und/oder Aromastoff-Depot geführt werden und wobei eine gewünschte Temperatur durch den Anteil an Verbrennungsgasen und ggf. durch das Mischungsverhältnis dieser Verbrennungsgase mit Umgebungsluft wählbar ist.

Ein solches Verfahren, vorzugsweise zum Inhalieren einer Nikotin-Aroma-Mixtur, wird ermöglicht durch die saubere Verbrennung eines Luft-Gasgemisches, d.h. die Abgase bestehen aus Stickstoff, gesundheitlich unbedenklichem Kohlenstoffdioxid, Wasser und ggf. Restsauerstoff und können so gefahrlos inhaliert werden. Dabei ist der Vorteil eines solchen Verfahrens im Gegensatz zu Verfahren worin die Wärme ausschließlich über Wärmetauscher an die Luft übertragen wird, dass prinzipbedingt eine Vorrichtung zur Durchführung dieses Verfahrens mit sehr viel kleineren Abmessungen bei höherem Wirkungsgrad und niedrigeren Herstellungskosten zu bewerkstelligen ist.

Weiterhin betrifft die Erfindung auch die Verwendung eines Ventils, wie bereits beschrieben, in einer mittels des durch den Sog eines Benutzers erzeugten Unterdrucks und/oder Luftstroms steuerbaren Inhaliervorrichtung oder in dem obenbeschriebenen Verfahren.

Folgende vorteilhafte Eigenschaften können durch die vorgestellten Produkte realisiert werden:
➢ Erscheinungsbild einer Zigarette
➢ Nikotin-Emission im Hauptstrom, wie bei einer Zigarette
➢ Geschmackserlebnis, wie beim Rauchen einer Zigarette
➢ Leichtes Kratzen bzw. Brennen im Mund- und Rachenraum, wie beim Rauchen einer Zigarette
➢ Handhabung ähnlich der einer Zigarette
➢ Geringe Herstellkosten, dadurch geringer Verkaufspreis der Hardware, vorzugsweise VK < 10 €
➢ Mindestens 50 %, vorzugsweise mindestens 95 % Schadstoffreduktion im Nebenstrom gegenüber einer herkömmlichen Zigarette (Abgas: nur Luft + Wasser und CO₂)
➢ Mindestens 20%, vorzugsweise mindestens 50 %, je nach Auswahl der Inhaltsstoffe sogar mindestens 90 % Schadstoffreduktion im Hauptstrom gegenüber einer herkömmlichen Zigarette (Abgas (+Luft) + z.B. Nikotin und Aromen)
➢ Eine gezielte Ausschließung von eventuell gesundheitsschädigenden Substanzen, z.B. Kanzerogene, sowie die Möglichkeit der gezielten Dosierung der Inhaltsstoffe und Emissionen unterhalb der zulässigen Grenzwerte.

### Kurze Beschreibung der Figuren

Im Folgenden werden nun Ausgestaltungen der Erfindung anhand der beiliegenden Figuren beschrieben.
Fig. 1 ist eine schematische Darstellung eines Systems bestehend aus einem Heizstab 2 und einem abnehmbaren Mundstück 3, sowie einer separaten Tankstation 1 zum Befüllen des Heizstabs 2.
Fig. 2 zeigt eine perspektivische Ansicht einer Ausführungsform a) einer Tankstation 1 mit eingelegtem Heizstab 2, b) mit geöffnetem Deckel 18 und herausgezogenem Heizstab 2, c) ein Mundstück 3 zum Aufstecken auf den Heizstab 2 und d) ein Heizstab 2 mit aufgestecktem Mundstück.
Fig.3 ist ein Längsschnitt einer Ausgestaltung des Heizstabs 2, bzw. des Heizstabs 2 mit aufgestecktem Mundstück 3.
   a) zeigt den Heizstab 2 beim Befüllen (Tankstation nicht dargestellt); wobei das Nachfüllventil 27 geöffnet, das Hauptventil 22 geschlossen und der Tank 21 befüllt wird,
   b) zeigt den Heizstab 2 mit aufgestecktem Mundstück 3 wodurch das Hauptventil 22 geöffnet wurde, das geöffnete Reglerventil 23 gibt den vollen Querschnitt frei: Betriebsflamme,
   c) zeigt den Heizstab 2 mit minimal offenem Ventil 23: Pilotflamme, sowie
   d) zeigt den Heizstab 2 mit durch Überhitzungsschutz 29 mittels Regleranschlag 290 geschlossenem Ventil 23, wobei der Regleranschlag 290 mehr Stellweg für das Reglerventil 23 freigibt und so den Strömungsquerschnitt vollständig verschließt: der Brenner 24 verlischt.

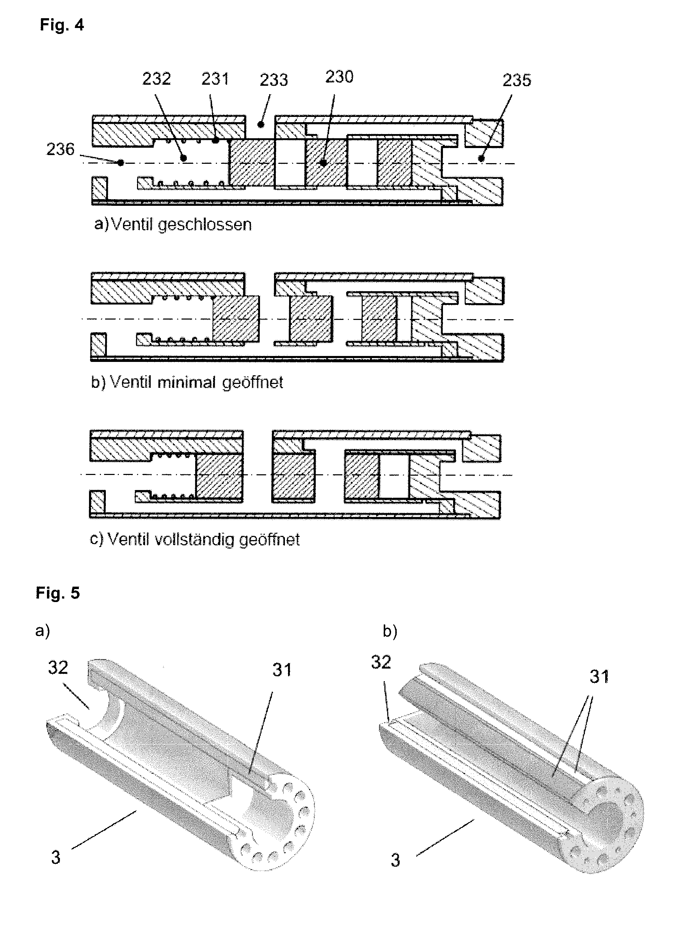

Fig. 4 zeigt einen Querschnitt einer zweiten Ausführungsform eines Reglerventils 23 im geschlossenem (aus), minimal offenem (Pilotflamme) und ganz offenem (Betriebsflamme) Zustand.
Fig. 5 zeigt a) ein Mundstück 3 mit einem Ein-Kammer-Depot 31 und Aerosolaustrittsöffnungen 32, sowie b) ein Mundstück 3 mit einem Mehr-Kammer-Depot 31.

Weitere Einzelheiten und Vorteile der Erfindung können der nachfolgenden ausführlichen Beschreibung möglicher Ausführungsformen der Erfindung anhand der beiliegenden Figuren entnommen werden.

### Beschreibung mehrerer Ausgestaltungen der Erfindung

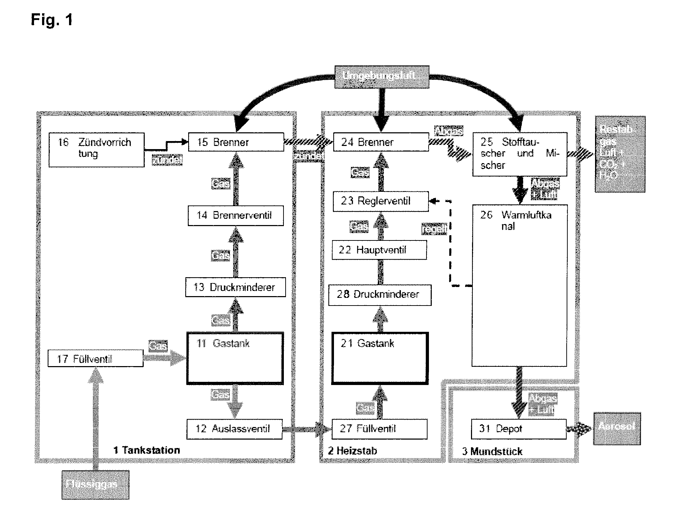

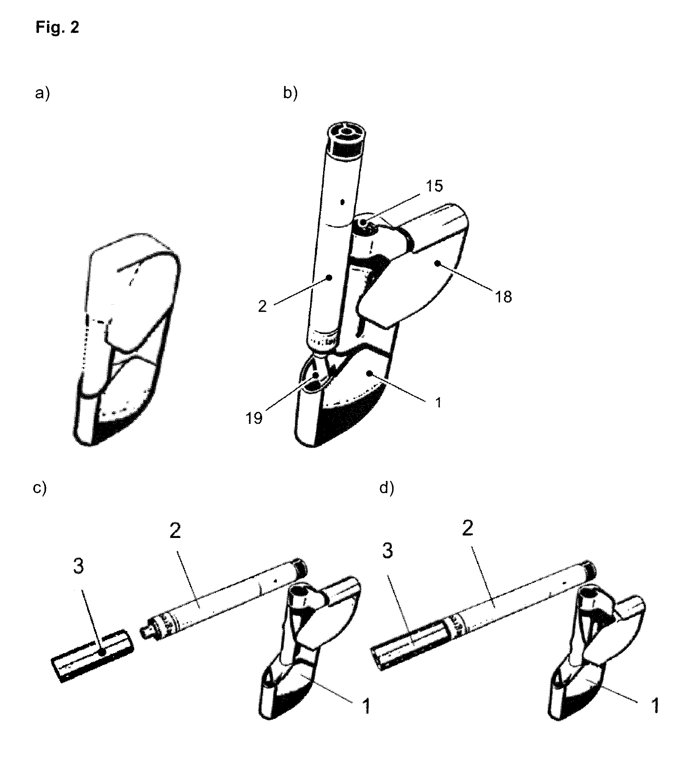

### 1. Ausgestaltungen der Tankstation 1 (s. Fig. 1 und 2)

Die Tankstation 1 dient prinzipiell zum Nachtanken, ggf. auch zum Aufbewahren und Entzünden des Heizstabs 2. Mit dem (eingelegten) Heizstab bildet sie die transportable Hardware des Produktes. In einer bevorzugten Ausgestaltung ist sie ein erweitertes Feuerzeug, welches den erfindungsgemäßen Heizstab 2 in sich aufnehmen und betanken kann.

### 1.1 Ausgestaltung T1 einer Tankstation

In einer ersten Ausgestaltung dient die Tankstation 1 ausschließlich dem Nachtanken des Heizstabs 2 und besteht deshalb nur aus einem Gastank 11 mit Nachfüllventil 17 zum Wiederbefüllen der Tankstation 1 und einem Auslassventil 12 zum Nachtanken des Heizstabs 2. Bevorzugt kann der Heizstab 2 zur Aufbewahrung bei Nichtgebrauch in der Tankstation 1 verbleiben. Zum Zünden des Heizstabs 2 muss entweder ein Zünder im Heizstab untergebracht sein (erhöhter Platzbedarf im Heizstab) oder ein zusätzliches Feuerzeug verwendet werden.

### 1.2 Ausgestaltung T2 einer Tankstation mit Zünder

Wie Ausgestaltung T1, jedoch mit zusätzlich integriertem Zündmechanismus 16 zum Zünden des Heizstabs 2. Gegenüber Ausgestaltung T1 entfällt jedoch die Notwendigkeit eines zusätzlichen Feuerzeugs (kein erhöhter Platzbedarf im Heizstab oder in der Tasche des Konsumenten).

### 1.3 Ausgestaltung T3 einer Tankstation mit Feuerzeug

Wie Ausgestaltung T1, jedoch mit zusätzlich integriertem Feuerzeug zum Zünden des Heizstabs 2. So kann die Tankstation 1 auch als herkömmliches Feuerzeug genutzt werden, was vor allem Kunden zugute kommt, die neben der rauchfreien Zigarette auch weiterhin herkömmliche Zigaretten konsumieren möchten.

### 1.3.1 Tankstationsausgestaltung T3 im Detail (s. Fig. 1 und 2)

### 1.3.1.1 Flüssiggastank

Der Flüssiggastank 11 ist das vom Bauraum her größte Bauteil der Tankstation 1 und die Energiequelle des Gesamtsystems rauchfreie Zigarette. Der mit handelsüblichem Feuerzeuggas wieder befüllbare Flüssiggastank 11 versorgt
➢ das in der Tankstation 1 integrierte Feuerzeug, wenn der Multifunktionsdeckel 18 in geöffnetem Zustand betätigt wird, (s. Fig. 2 b)
➢ den Heizstab 2 in der Heizstabaufnahme 19, wenn der Multifunktionsdeckel 18 in geschlossenem Zustand bei eingelegtem Heizstab 2 gedrückt wird (s. Fig. 2 a).

### 1.3.1.2 Integriertes Feuerzeug

Das integrierte Feuerzeug ist z.B. ein Vormischbrenner 15 mit Piezozündung 16, wie bei handelsüblichen Sturmfeuerzeugen bekannt. Gaszufuhr und Zündung werden über den Multifunktionsdeckel 18 in geöffnetem Zustand getätigt. Das Feuerzeug dient zum Entzünden des Heizstabs 2. Weiter kann es auch als herkömmliches Feuerzeug verwendet werden.

### 1.3.1.3 Multifunktionsdeckel

Der Deckel 18 der Tankstation 1 ist durch seine Formgebung und Kinematik bevorzugt so gestaltet, dass er vier verschiedene Funktionen erfüllt: Heizstab betanken, Heizstab arretieren, Hitzeschild für Heizstabbrenner bereitstellen und Feuerzeug einschalten. Prinzipiell lassen sich auch alle Funktionen des Multifunktionsdeckels durch mehrere Bauteile in verschiedensten Varianten lösen. Der Lösungsansatz "Multifunktionsdeckel" wird jedoch aufgrund des hohen Integrationsgrades von Funktionen als eleganteste und wahrscheinlich kostengünstigste Variante bevorzugt.

Deshalb sollen an dieser Stelle alle vier Funktionen durch den Deckel übernommen werden.

Funktion 1: Heizstab 2 betanken: In geschlossenem Zustand umschließt der Deckel 18 die Spitze des Heizstabes 2. Drückt man den Deckel herunter wird auch der Heizstab 2 in seine Aufnahme gedrückt, so dass das Ventil 12 der Tankstation 1 am Boden der Aufnahme gasdicht auf das Einlassventil 27 des Heizstabs 2 gepresst wird. Durch das Anpressen öffnen beide Ventile und der Heizstab 2 wird von der Tankstation 1 solange mit Gas gefüllt bis der Druck zwischen Tankstation 1 und Heizstab 2 ausgeglichen ist oder der Deckel 18 losgelassen wird. Der Druckausgleich wird vorzugsweise von einem zischenden Geräusch begleitet. Hört das Geräusch auf, ist der Heizstab voll betankt.

Funktion 2: Heizstab 2 arretieren: Der Rastmechanismus des Deckels 18 ist so ausgelegt, das er mit oder ohne eingelegten Heizstab 2, durch Überdrücken der Tankposition, je nach vorigem Zustand, ein- oder ausrastet. Mit eingelegtem Heizstab 2 wird synchron zum Überdrücken des Deckels 18 der Heizstab 2 betankt.

Funktion 3: Hitzeschild für Brenner: Der Deckel 18 ist so beschaffen, dass er über das nach Gebrauch heiße Ende des Heizstabs 2 (Brennerspitze) geschoben und/oder geklappt wird. Er bildet einen Hitzeschutz, der bei geschlossenem Deckel 18 einen Kontakt mit der Brennerspitze des Heizstabs 2 vermeidet. Weiter ist er in seinen wärmeleitenden Eigenschaften so ausgelegt, dass zum einen die Brennerspitze schnellstmöglich abkühlen kann und zum anderen auf der Deckelaußenseite keine für Menschen unangenehme Temperatur auftritt.

Funktion 4: Feuerzeug einschalten: Im voll geöffneten Zustand wird durch ein Überdrücken des Multifunktionsdeckels 18 das in der Tankstation 1 integrierte Feuerzeug betätigt, d.h. die Gaszufuhr wird freigeschaltet und der Zünder 16 des Feuerzeugs in einer Bewegung getätigt.

### 1.3.1.4 Heizstabaufnahme

Die Aufnahmetasche 19 für den Heizstab 2 ist bevorzugt so gestaltet, dass der Heizstab 2 nur mit seinem mundstückseitigen Ende ohne aufgestecktes Mundstück 3 eingeschoben werden kann.

### 1.3.1.5 Auslassventil

Auf dem Boden der Aufnahmetasche 19 befindet sich das Auslassventil 12 der Tankstation 1, welches z.B. durch Herunterdrücken des Multifunktionsdeckels 18 bei eingelegtem Heizstab 2 mit diesem gasdicht verbunden wird. Das Auslassventil 12 ist dabei bevorzugt so gestaltet, dass es durch Niederdrücken sich selbst und das Einlassventil 27 des Heizstabs 2 öffnet, so dass Gas aus der Tankstation 1 in den Heizstab 2 strömen kann.

### 2. Heizstab (s. Fig. 1, 2 und 3)

Den nachfolgenden Heizstabausgestaltungen ist gemein, dass sie Luft zum Bilden eines mit Nikotin und Aromen angereicherten Aerosols mittels Verbrennung von Flüssiggas erwärmen. Die Dimensionen des Heizstabs sollten die einer herkömmlichen Zigarette (ohne Filter) nicht signifikant überschreiten.

### 2.1 Ausgestaltung H1 eines Heizstabs mit Wärmetauscher

Das heiße Abgas des Gasbrenners 24 im Heizstab 2 wird durch einen Wärmetauscher 25 geleitet, wo es einen Teil seiner Energie in Form von Wärme an die Inhalationsluft überträgt. Abgas und Inhalationsluftstrom sind so vollständig getrennt.

### 2.2 Ausgestaltung H2 eines Heizstabs mit Stofftauscher

Anstelle eines Wärmetauschers wird in diesem Fall dem Brenner 24 ein Stofftauscher und -mischer 25 nachgeschaltet. D.h. die Umgebungsluft wird mit heißem Abgas vermischt. Das Mischverhältnis und somit die Inhalationslufttemperatur am Stofftauscherausgang hängt von der Auslegung des Stofftauschers 25 ab (Verhältnis der Einströmquerschnitte: Abgasseite zu Umgebungsluftseite).

Der Stofftauscher 25 hat einen wesentlich besseren Wirkungsgrad bei der Ausnutzung der Verbrennungswärme als der Wärmetauscher und ist so je nach geforderter Größe des Heizstabs 2 oder der zu verflüchtigen Mixturen bevorzugt einzusetzen.

### 2.2.1 Bevorzugtes Heizstabkonzept H2 im Detail

### 2.2.1.1 Hülse

Die Hülse 20 des Heizstabes 2 kann aus einem oder mehreren zylindrischen Teilen bestehen. Das Äußere der Hülse 20 verleiht dem Heizstab 2 sein zigarettenartiges Aussehen und bildet die Haltefläche für den Konsumenten. Sie ist der Hauptkomponententräger des Heizstabs 2. Auf Höhe des Minitanks 21 ist die Hülse 20 innen so geformt, dass zum einen Luftströmungskanäle zwischen Hülse 20 und Minitank 21 entstehen und zum anderen der Minitank 21 axial in ihr verschiebbar ist. Am mundstückseitigen Ende ist sie durch eine Kappe so geschlossen, dass der Minitank 21 in seiner axialen Bewegung einen Anschlag hat. Für den Füllstutzen 27 des Minitanks 21 ist in dieser Kappe eine Öffnung vorhanden. Radial um diese Öffnung sind ringförmig kleine Luftströmungsöffnungen angebracht. An der Außenfläche der Kappe ist eine Dichtung aufgebracht, die beim Aufschrauben eines Mundstückes auf den Bajonettverschluss des Minitanks 21 ein Einströmen von Fremdluft verhindert, so dass beim Saugen am aufgeschraubten oder aufgesteckten Mundstück 3 die Luft durch den Heizstab 2 zwangsgeführt wird.

Die Hülse 20 ist Komponententräger und bevorzugt Funktionspartner für folgende weitere Baugruppen und Bauteile:
a. Minitank-Hauptventilhalter 22
b. Regelventil 23
c. Brenner 24

### 2.2.1.2 Minitank

Der Minitank 21 ist die Energiequelle für den Brenner 24 im Heizstab 2 und so dimensioniert, dass das in ihm gespeicherte Flüssiggas für den Konsum eines Mundstücks 3 ausreicht. Die Dauer eines Mundstückkonsums soll der eines Zigarettenkonsums so nah wie möglich kommen, d.h. je nach Gebrauch 3 - 5 min sein. An seinem mundstückseitigen Ende befindet sich das Einlassventil 27 zum Betanken des Heizstabs 2 durch die Tankstation 1. Dieses Ende ist z.B. so geformt, dass ein Bajonettverschluss mit dem Mundstück gebildet wird. Der Minitank 21 ist im Heizstab axialbeweglich untergebracht, so dass beim Aufschrauben/stecken eines Mundstücks 3 der Tank 21 zum Mundstück 3 hin bewegt wird. Am brennerseitigen Ende des Minitanks 21 ist das Hauptventil 22 untergebracht, ggf. mit einem zwischengeschalteten Druckminderer 28. Das Hauptventil 22 entspricht im Prinzip einem herkömmlichen Auslassventil eines Feuerzeugs. Im Gegensatz zum Feuerzeug wird das Ventil aber nicht durch Fixieren des Tanks und ziehen der Ventildüse sondern durch Halten der Ventildüse und Ziehen des Tanks 21 betätigt. Wird also ein Mundstück 3 auf den Heizstab 2 aufgeschraubt, gibt das Hauptventil 22 die Gaszufuhr vom Tank 21 zum Reglerventil 23 frei. Zwischen Tank 21 und mundstückseitiger Kappe der Hülse 20 kann zusätzlich ein kleines Federelement angebracht werden, falls das Hauptventil 22 keine ausreichende Kraft zum Zurückziehen des Tanks 21 und somit zum Schließen hat, wenn das Mundstück entfernt wird.

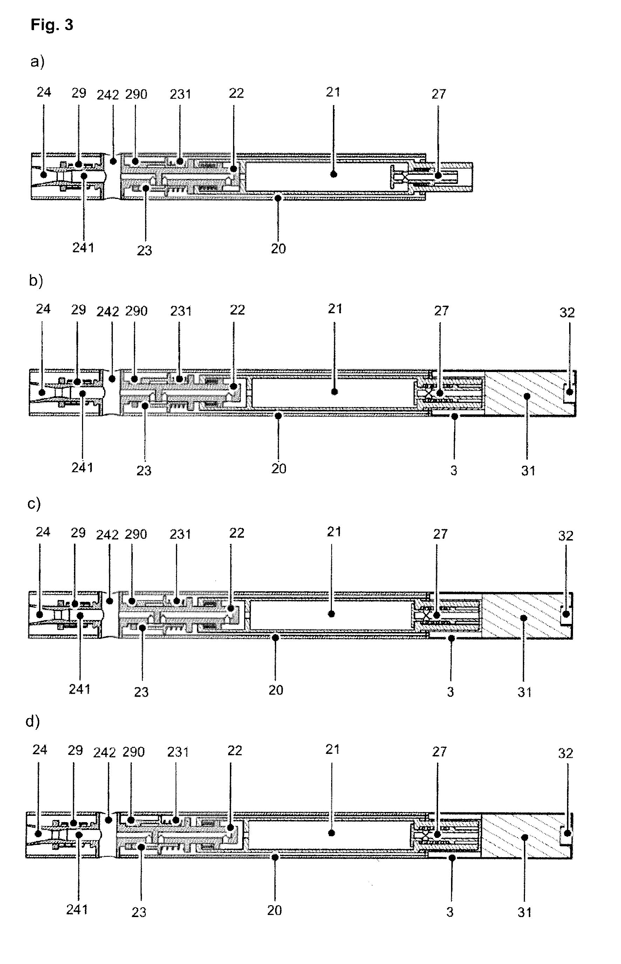

### 2.2.1.3 Reglerventil (s. Fig. 3 und 4)

### 2.2.1.3.1 Ausgestaltung R1 eines Reglerventils

Das Reglerventil 23 steuert die Gas und Luftzufuhr für den Brenner 24 in Abhängigkeit von der Strömung der Inhalationsluft innerhalb der Hülse 20. Es ist axial beweglich auf dem Mittelteil der Düse zwischen dem Hauptventil 22 des Minitanks 21 und dem Brenner 24 angeordnet. Außen schließt das Reglerventil 23 luftdicht mit der Hülse 20 ab. In radialer Richtung ist das Reglerventil 23 z.B. mit kleinen Bohrungen für die Luftversorgung des Brenners 24 und Einkerbungen auf der Innenseite zur Regelung des Gasstroms versehen.

In axialer Richtung ist das Reglerventil 23 mit kleinen Bohrungen versehen, so dass das Reglerventil 23 z.B. als Drossel im Luftstrom fungiert. Je nach Druckdifferenz vor und hinter dem Ventil 23 wird es entweder zum Mundstück 3 hingezogen oder von ihm weggedrückt. Das Reglerventil 23 kann drei relevante Positionen mit folgenden Eigenschaften einnehmen:

- Aus: Der Regelkörper des Reglerventils 23 befindet sich in der von Mundstückseite aus vordersten Position. Luft- und Gaszufuhr sind vollständig getrennt. In dieser Position ist der Regelkörper durch Kraftschluss so arretiert, dass eine durch einen Volumenstrom von ca. 17,5 ml/s auf die Drossel erzeugte Kraft in Richtung Mundstück 3 (durchschnittlicher Sog eines Konsumenten) zum Öffnen des Ventils 23 benötigt wird. Zum Schließen muss die gleiche Kraft in entgegengesetzter Richtung aufgebracht werden.

- Pilotflamme: Der Regelkörper des Reglerventils 23 befindet sich in Mittelstellung (diese kann auch exzentrisch auf dem Gesamtstellweg liegen), wodurch gerade soviel Gas und Luft das Ventil passieren, dass die Flamme im Brenner 24 nicht erlischt.

- Betriebsflamme: Der Regelkörper des Reglerventils 23 ist aus der Mittelstellung durch Sog am Mundstück 3 in Richtung desselben bewegt worden und gibt nun ausreichend Luft und Gas für den Brenner 24 frei, sodass dieser ausreichend Brenngas zur Erwärmung der Inhalationsluft abgibt. Wird der Regelkörper über die Mittelstellung (Pilotflamme) hinaus in Richtung Mundstück 3 bewegt, so drückt er eine Feder 231 zusammen. Die Feder 231 ist so ausgelegt, dass sie, ohne Sog am Mundstück, den Regelkörper des Ventils 23 in die Mittelstellung, aber nicht darüber hinaus, drückt.

Durch verschiedene Geometrien der Öffnungen am Ventil 23 sind verschiedene Kennfelder für den Brenner darstellbar.

### 2.2.1.3.2 Ausgestaltung R2 eines Reglerventils (s. Fig. 4)

In einer möglichen Variante des Reglerventils, wird der Regelkörper des Ventils 23 nicht durch den Luftstrom im Mantel, sondern durch den vom Mantelstrom ausgehenden Unterdruck an der Brennerdüse getätigt. Der Mantelstrom wird durch ein Saugen des Konsumenten am Mundstück erzeugt.

In Fig. 4 a) ist das Reglerventil 23 geschlossen, sowohl der Gaseinlass 236 als auch der Umgebungslufteinlass 233 sind zu (aus).

In Fig. 4c) bei angelegtem Unterdruck in der Unterdruckkammer 232 wird der Regelkörper 230 entgegen der Kraft der Rückstellfeder 231 gezogen und gibt sowohl den Gaseinlass 235 als auch den Umgebungslufteinlass 233 frei: das Luft-Gasgemisch strömt über den Gemischauslass 236 in den Brenner (Betriebsflamme).

In Fig. 4 b) wird nachdem kein Unterdruck mehr in der Unterdruckkammer 232 herrscht der Regelkörper 230 durch die Rückstellfeder 231 in eine minimal offenen Zustand gebracht, wobei sowohl der Gaseinlass 236 als auch der Umgebungslufteinlass 233 gerade so weit geöffnet sind, dass die Flamme im Brenner nicht erlischt (Pilotflamme). 2.2.1.4 Brenner

Der Brenner 24 ist bevorzugt ein Flüssiggas-Vormischbrenner, d.h. das brennbare Gemisch aus Gas und durch die Lufteingänge 242 einströmende Luft wird bereits vor dem Düsenaustritt in der Mischkammer 241 gebildet, sodass eine vollständige Verbrennung des Flüssiggases gewährleistet wird. Zu dieser Art von Brennern werden z.B. Nainen-, Ikari- oder Matrix-Brenner gezählt und sind teilweise aus Sturmfeuerzeugen bekannt.

Typische Nainen-Brenner haben ca. 0,5 cm oberhalb des Düsenauslaßes eine ringförmige Öffnung mit einem so genannten Reaktionsgitter, das durch die Zündflamme erhitzt wird und eine ständige Wiederentzündung des hindurch strömenden Gases bewirkt. Nainen-Brenner bilden oberhalb des Reaktionsgitters eine kegelförmige, kurze, nichtleuchtende, blaue Flamme, die am Fuß einen Durchmesser gleich der Ringöffnung hat.

Zur Verbesserung der Lastwechseleigenschaften zwischen Pilotflamme und Betriebsflamme kann auch die Verwendung eines dreidimensionalen Reaktionsgitters vorgesehen werden.

Weiterhin ist der Brenner 24 optional mit einem Überhitzungsschutz, z.B. in Form eines Bimetallreglers 29 ausgestattet, der z.B. durch Ausdehnung den Regleranschlag 290 steuert und so das Reglerventil 23 bei Überhitzung schließt.

### 2.2.1.5 Wärme- und/oder Stoffübertrager, bzw. -tauscher

Der Wärme- und/oder Stoffübertrager 25 überträgt je nach Auslegung Wärme und/oder Abgas vom Brenner 24 zur Inhalationsluft. Falls ein reiner Wärmeübertrager je nach Anforderung eventuell nicht den, aufgrund der geringen Abmaße des Heizstabs 2, nötigen Wirkungsgrad aufweist, kann auch ein reiner Stoffübertrager oder ein kombinierter Wärme- und Stoffübertrager Verwendung finden.

Ein Stoffübertrager ist so gestaltet, dass beim Saugen am Mundstück Abgas mit Umgebungsluft so vermischt wird, dass das entstehende Gemisch die richtige Temperatur zum Lösen der Mixtur aus dem Mundstück besitzt. Dabei muss der Temperaturverlust beim Durchströmen des Heizstabs mit eingerechnet werden.

### 3 Software

Als Software werden alle Bauteile und Stoffe bezeichnet, die nach einmaligem Gebrauch ausgetauscht werden müssen. Die Software besteht aus dem Mundstück 3 und der darin als Depot 31 aufgebrachten Mixtur.

### 3.1 Mundstück (Fig. 1, 2, 3 und 5)

Das Mundstück 3 entspricht in seiner Größe in etwa einem Zigarettenfilter, hat jedoch im Gegensatz zu diesem keine Filterfunktion. Im Gegensatz zu einem Filter soll das Mundstück keine Substanzen aus der Inhalationsluft herausziehen, sondern der Inhalationsluft Stoffe zusetzen. Alle Teile des Mundstücks 3 bestehen bevorzugt zu 100 % aus biologisch abbaubaren Materialien.

### 3.1.1 Hülle

Die ein- oder mehrteilige Hülle des Mundstücks 3 ist Haltefläche für den Konsumenten und Kontaktfläche mit dem Mund des Konsumenten. Sie ist bevorzugt ein zylindrischer gas- und flüssigkeitsdichter Hohlkörper mit Öffnungen an beiden Enden. Das heizstabseitige Ende ist so geformt, dass die Inhalationsluft ungestört aus den Warmluftausgängen des Heizstabs 2 in das Mundstück 3 strömen kann und dass eine gasdichte Verbindung durch Anpressen der Stirnseite an die Dichtung des Heizstabs 2 entsteht. Das Anpressen wird z.B. durch das in der Hülle ausgeformte Gegenstück zum Bajonettverschluss des Heizstabs o.ä. ermöglicht.

### 3.1.2 Depot

Das Depot 31 ist im Prinzip koaxial zur Hülle innerhalb dieser angeordnet und dient der Speicherung der Wirk- und/oder Aromastoff-Mixtur. Die Affinität des Depotmaterials zur Mixtur muss so hoch sein, dass auch bei Lagerbedingungen mit erhöhter Temperatur so gut wie keine Mixtur vom Depot abgegeben wird. Bei Erreichen der Betriebstemperatur der Inhalationsluft (Warmluft), die das Mundstück durchströmt muss das Depot 31 jedoch die Mixtur in gefordertem Umfang freisetzen und als Aerosol über die Aerosolaustrittsöffnung 32 dem Benutzer zukommen lassen.

Das Depot 31 im Mundstück 3 kann bei Bedarf auch aus mehreren Kammern bestehen (s. Fig. 5 b).

Um die Unterschiede der Verdampfungstemperaturen von z.B. den Aromen und Nikotin zu berücksichtigen, ist es eventuell notwendig diese in getrennten Depots 31 mit unterschiedlichen Strömungsquerschnitten und/oder unterschiedlichen Eigenschaften des Depotmaterials (Wärmekapazität, Affinität zur aufgegebenen Mixtur, usw.) unterzubringen.

Durch eine Einrastposition z.B. des Bajonettverschlusses zwischen Mundstück 3 und Heizstab 2 können die Durchströmungsöffnungen von Mundstück 3 und die Warmluftausgänge des Heizstabs 2 in die richtige Position gebracht werden.

### 3.1.3 Mundstückversiegelung

Aus hygienischen Gründen und Gründen der Halt- und Lagerbarkeit kann das Mundstück 3 auch versiegelt werden. Eine Mundstückversiegelung kann z.B. aus zwei kleinen gas- und flüssigkeitsdichten Folien bestehen, die auf die Stirnseiten des Mundstücks geklebt und vor Gebrauch entfernt werden. Zum einfachen Entfernen können die Folien zusätzlich mit einer kleinen Lasche versehen werden.

### 3.2 Wirk- und/oder Aromastoff-Mixtur

Die Mixtur ist der Hauptbestandteil der Rauchsimulation, da sie alle gewünschten Stimulantien enthält, die durch ausreichende Temperatur freigesetzt werden. Die möglichen Inhaltsstoffe gliedern sich in folgende Gruppen.

### 3.2.1 Wirkstoffe

Die Wirkstoffe bestehen in erster Linie aus Nikotin und dessen Derivaten, obwohl andere Stimulantien ebenfalls oder stattdessen eingesetzt werden können, z.B. Koffein und/oder Taurin. Die Menge an Wirkstoff in der Mixtur kann sehr einfach bei der Herstellung dosiert werden und könnte z.B. weniger Nikotin als eine herkömmliche Zigarette enthalten, jedoch bei erreichen der Betriebstemperatur die gleiche Menge, wie eine Zigarette freisetzen. Weiterhin können Vitamine und Mineralien zugesetzt werden.

### 3.2.2 Aroma- oder Geschmacksstoffe

Die Geschmacksstoffe sind hauptsächlich Aromen, die laut Aromenverordnung zugelassen sind. Auch z.B. Zucker kann als Geschmacksstoff zugesetzt werden.

### 3.2.3 Reizstoffe

Die Reizstoffe dienen der Imitation des "Kratzens" im Rachenraum, welches beim Rauchen entsteht. Hier kann z.B. Capsaicin (erzeugt die Schärfe in Lebensmitteln) und/oder andere Reizstoffe in unbedenklichen Mengen Verwendung finden.

### 3.2.4 Verflüchtigungsvermittler

Verflüchtigungsvermittler wie Ethanol, Wasser, Polyole oder ähnliche können zur besseren Verdampfung der Mixtur zugesetzt werden.

### 3.2.5 Stabilisatoren

Um die Lagerfähigkeit zu erhöhen und/oder unerwünschte Reaktionen zwischen den Bestandteilen der Mixtur zu verhindern können auch diverse Stabilisatoren angewendet werden.

### 3.2.6 Weitere Zusatzstoffe

Die Verwendung weiterer Zusatzstoffe ist möglich soweit sie gesundheitlich unbedenklich in den verwendeten Mengen für den Konsumenten sind.

## Patentansprüche

1. Vorrichtung in Form und Abmessungen einer Zigarette oder Zigarre zur Abgabe eines inhalierbaren Aerosols, umfassend
➢ ein einen Wirk- und/oder Aromastoff-Depot (31) enthaltendes Mundstück (3),
➢ einen Heizstab (2) umfassend
eine Gehäusehülse mit einem oder mehreren Lufteingängen und
einem oder mehreren mundstückseitigen Warmluftausgängen,
ein Füllventil (27) zum Befüllen eines Gastanks (21) mit einem brennbaren Gas, vorzugsweise Propan- oder Butangas,
ein Reglerventil (23) zur gesteuerten Abgabe des Gases aus dem Gastank (21) an einen Brenner (24) und
einen Wärme- und/oder Stofftauscher (25) zur Erwärmung der Luft durch die mittels des Brenners (24) erzeugten Hitze,
wobei das Mundstück (3) mit dem Heizstab (2) lösbar verbunden ist und die Steuerung des Reglerventils (23) mittels des durch den Sog eines Benutzers am Mundstück (3) erzeugten Unterdrucks und/oder Luftstroms erfolgt.

2. Ventil für Inhaliervorrichtung, steuerbar mittels eines durch den Sog eines Benutzers erzeugten Unterdrucks und/oder Luftstroms, umfassend einen Regelkörper, sowie Mittel zur Erzeugung einer Rückstellkraft, wobei der Regelkörper einen ersten geschlossenen Zustand, einen zweiten offenen Zustand, sowie einen dritten minimal offenen Zustand aufweist, wobei der Regelkörper mittels des erzeugten Unterdrucks und/oder Luftstroms aus dem ersten geschlossenen Zustand entgegen einer Rückstellkraft in den zweiten offenen Zustand versetzbar ist und die Mittel zur Erzeugung einer Rückstellkraft derart gewählt sind, dass sie den Regelkörper nur in den dritten minimal offenen Zustand zurücksetzen können.

3. Ventil nach Anspruch 2, wobei der Regelkörper in dem zweiten offenen Zustand einen Strömungsquerschnitt freigibt der abhängig vom Unterdruck und/oder Luftstrom variabel zwischen einem minimal offenen bis ganz offenen Zustand steuerbar ist.

4. Vorrichtung nach Anspruch 1, wobei das Reglerventil (23) ein Ventil nach einem der Ansprüche 2 oder 3 ist.

5. Vorrichtung nach Anspruch 1 oder 4, umfassend zusätzlich ein Hauptventil (22) zwischen Gastank (21) und Reglerventil (23) welches durch Verbinden des Mundstücks (3) mit dem Heizstab (2) geöffnet und durch Ablösen des Mundstücks (3) wieder geschlossen wird.

6. Vorrichtung nach einem der Ansprüche 1, 4 oder 5, wobei ein oder mehrere zu dem oder den Warmluftausgängen führende Warmluftkanäle koaxial um den Gastank (21) angeordnet sind.

7. Vorrichtung nach einem der Ansprüche 1 oder 4 bis 6, in der das Wirk- und/oder Aromastoff-Depot (31) als Wirkstoff Nikotin und ggf. Aromastoffe, sowie ggf. weitere Zusatz- und Hilfsstoffe, wie Reizstoffe, Stabilisatoren, Verflüchtigungsvermittler, usw. enthält.

8. Vorrichtung nach Anspruch 7, wobei der oder die Wirkstoff(e), ggf. Aromastoff(e), sowie ggf. weitere Zusatz- und Hilfsstoffe in mehreren Kammern einzeln und/oder gemischt vorliegen.

9. Vorrichtung nach einem der Ansprüche 1 oder 4 bis 8, in der ein Stofftauscher (25) eingesetzt wird und ein Kohlenmonoxid-Oxydationskatalysator, z.B. ein Hopkalit-Katalysator, diesem Stofftauscher (25) nachgeschaltet ist.

10. Tankstation (1) zum Befüllen des Heizstabs (2) einer Vorrichtung nach einem der Ansprüche 1 oder 4 bis 9 , umfassend
einen Gasvorratstank (11) dessen Volumen das mehrfache des Volumens des Gastanks (21) des Heizstabs (2) beträgt und
ein form- und kraftschlüssig mit dem Füllventil (27) des Heizstabs (2) verbindbares Auslassventil (12).

11. Tankstation nach Anspruch 10, wobei Mittel zur Aufbewahrung eines Heizstabs (2) vorgesehen sind, umfassend eine das Füllventil zu mindest teilweise umschließende Aufbewahrungstasche (B1) zur Aufnahme eines mundstückseitigen Teils des Heizstabs (2), sowie einen umsetzbaren Deckel (B2) der in einer geschlossenen Stellung die entgegengesetzte Seite des Heizstabs (2) überdeckt.

12. Tankstation nach Anspruch 10 oder 11, welche zusätzlich eine Zündvorrichtung (16) enthält.

13. Tankstation nach Anspruch 12, welche weiterhin ein Brennerventil (14) und einen Brenner (15) umfasst.

14. Tankstation nach einem der Ansprüche 10 bis 13, welche weiterhin ein Füllventil (17) zum Befüllen des Gasvorratstanks (11) umfasst.

15. Kit umfassend eine Tankstation (1) nach einem der Ansprüche 10 bis 14 und einen Heizstab (2) wie in einem der Ansprüche 1 oder 4 bis 9 beschrieben, gegebenenfalls mit einer Vielzahl an Mundstücken (3) wie in einem der Ansprüche 1 oder 4 bis 9 beschrieben.

16. Verfahren zur Verflüchtigung von Wirk- und Aromastoffen zwecks Inhalation, wobei Verbrennungsgase eines brennbaren Gases teilweise oder vollständig, gegebenenfalls vermischt mit Umgebungsluft, durch einen Wirk- und/oder Aromastoff-Depot geführt werden und wobei eine gewünschte Temperatur durch den Anteil an Verbrennungsgasen und ggf. durch das Mischungsverhältnis dieser Verbrennungsgase mit Umgebungsluft wählbar ist.

17. Verwendung eines Ventils gemäß Anspruch 3 oder 4 in einer mittels des durch den Sog eines Benutzers erzeugten Unterdrucks und/oder Luftstroms steuerbaren Inhaliervorrichtung oder in einem Verfahren gemäss Anspruch 16.
